# EUROPÄISCHE PATENTSCHRIFT

(11) **EP 1 252 164 B1**
(45) Veröffentlichungstag und Bekanntmachung des Hinweises auf die Patenterteilung: **24.03.2004**
(21) Anmeldenummer: 01905691.0
(22) Anmeldetag: 26.01.2001
(51) Int. Cl.: C07D 487/04, A61K 31/40, A61P 21/00

(54) **VERFAHREN ZUR HERSTELLUNG VON 6-(4-CHLORPHENYL)-2,2-DIMETHYL-7-PHENYL-2,3-DIHYDRO-1H-PYRROLIZIN-5-YL-ESSIGSÄURE**
METHOD FOR PRODUCING 6-(4-CHLOROPHENYL)-2,2-DIMETHYL-7-PHENYL-2,3-DIHYDRO-1H-PYRROLIZINE-5-YL ACETIC ACID
PROCEDE DE PREPARATION D'ACIDE 6-(4-CHLOROPHENYL)-2,2-DIMETHYL-7-PHENYL-2,3-DIHYDRO-1H-PYRROLIZIN-5-YL-ACETIQUE

(30) Priorität: 28.01.2000 MX 0001047
(43) Veröffentlichungstag der Anmeldung: 30.10.2002
(73) Patentinhaber: MERCKLE GMBH, D-89079 Ulm (DE)
(72) Erfinder: KAMMERMEIER, Thomas, 89073 Ulm (DE); LAUFER, Stefan, 89143 Blaubeuren (DE); MERCKLE, Philipp, 89143 Blaubeuren-Weiler (DE); STRIEGEL, Hans-Günter, 89134 Blaustein (DE)
(74) Vertreter: Kinzebach, Werner, Dr.
(86) Internationale Anmeldenummer: PCT/EP2001/000852
(87) Internationale Veröffentlichungsnummer: WO 2001/055149

(56) Entgegenhaltungen:
- WO-A-95/32971
- COSSY J ET AL: "Synthetic Studies Towards ML-3000 A Concise Synthesis of This Non-Steroidal Anti-Inflammatory Drug" TETRAHEDRON,NL,ELSEVIER SCIENCE PUBLISHERS, AMSTERDAM, Bd. 55, Nr. 16, 16. April 1999 (1999-04-16), Seiten 5145-5156, XP004161093 ISSN: 0040-4020

## Beschreibung

Die vorliegende Erfindung betrifft ein Verfahren zur Herstellung von 6-(4-Chlorphenyl)-2,2-dimethyl-7-phenyl-2,3-dihydro-1H-pyrrolizin-5-yl-essigsäure (ML 3000), sowie ein neues Polymorph von ML 3000, das als Polymorph A bezeichnet wird.

ML 3000 ist ein vielversprechender Hemmstoff der Cyclooxygenase und 5-Lipoxygenase und eignet sich somit zur Behandlung von Erkrankungen des rheumatischen Formenkreises und zur präventiven Behandlung von allergisch induzierten Erkrankungen, siehe dazu z.B. Drugs of the Future 1995, 20 (10):1007-1009. In dieser Publikation findet sich auch ein möglicher Weg zur Herstellung. Weitere Herstellungsmöglichkeiten sind beschrieben in EP-A-397175, WO95/32970, WO95/32971, WO95/32972, Archiv der Pharmazie 312, 896-907 (1979); und 321, 159 - 162 (1988), J. Med. Chem. 1994 (37), 1894-1897, Arch. Pharm. Pharm. Med. Chem. 330, 307-312 (1997). Bei allen diesen Synthesen erfolgt der Aufbau des Pyrrolizingrundgerüstes nach der in der Formelübersicht dargestellten Methode:

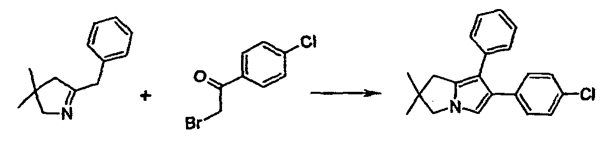

Die Umsetzung wird in Methylenchlorid, Ethanol oder Diethylether durchgeführt. Der bei der Reaktion gebildete Bromwasserstoff wird durch Zusatz wässriger Natriumbicarbonat-Lösung abgefangen.

Die Einführung des Essigsäurerestes in Position 5 erfolgt dann durch Umsetzung mit Diazoessigester oder Oxalesterchlorid und anschließende Verseifung bzw. Verseifung und Reduktion der Ketogruppe mit Hydrazin.

Arch. Pharm. 312, 896-907 (1979) beschreibt folgende Umsetzung:

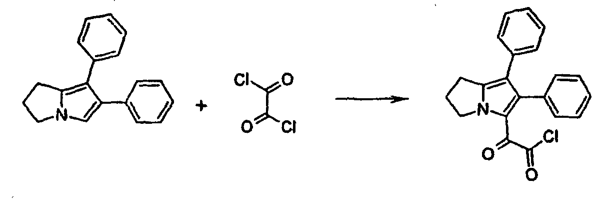

Die Umsetzung wird in Benzol als Lösungsmittel durchgeführt. Die COCOCI-Gruppierung wird dann jedoch nicht in die Essigsäure-Gruppe überführt, sondern mit Diethylamin umgesetzt.

WO95/32970, WO95/32971 und WO95/32972 beschreiben die Einführung des Essigsäurerestes in Verbindungen, die zu ML-3000 strukturverwandt sind, durch Umsetzung dieser Pyrrolizin-Verbindungen mit Oxalylchlorid oder Ethyloxalylchlorid und anschließende Reduktion mit Hydrazin und Kaliumhydroxid (Huang Minion-Variante der Wolff-Kishner-Reduktion). Nähere Angaben zur experimentellen Durchführung finden sich nur im Beispiel 5C der WO95/32971. Danach erfolgt die Umsetzung der Pyrrolizin-Verbindung mit Oxalylchlorid in THF. Zum Reaktionsprodukt gibt man Wasser und Hydrazinhydrat und, destilliert das Tetrahydrofuran ab, versetzt den Rückstand mit Diethylenglycol und mit Kaliumhydroxyd und erhitzt auf 140 °C unter gleichzeitigem Entfernen des Wassers. Anschließend versetzt man die Reaktionsmischung mit Wasser, säuert an und nimmt die ausgeschiedene Carbonsäure in Diethylether auf Das Produkt wird gereinigt indem die etherische Lösung über einem Trocknungsmittel, wie wasserfreiem Natriumsulfat oder Magnesiumsulfat, einige Zeit gerührt und stehen gelassen, anschließend das mit Wasser gesättigte Sulfat abfiltriert und schließlich der Ether in der Wärme abgedampft wird. Die aus der Mutterlauge beim Konzentrieren kristallierende Substanz wird gesammelt und getrocknet.

Für die industrielle Herstellung von ML 3000 ist die Einführung des Essigsäure-Restes mit Oxalylchlorid bevorzugt. Allerdings hat sich gezeigt, dass nach der obigen Umsetzung in den bisherigen Verfahren der Isolierung und Reinigung der Rohware die Ausbeute stark abfällt und eine Reihe von Zersetzungsprodukten im Reinigungsschritt und noch während der Trocknung neugebildet werden, sodass eine weitere aufwendige Reinigung des ML 3000, z.B. durch Umkristallisation erforderlich ist um Pharmaqualität zu erhalten.

In den bereits erwähnten Herstellungsverfahren wurden zu ML 3000 strukturanaloge Verbindungen nach folgenden Methoden gereinigt und kristallisiert.

In J. Med. Chem. 1994, 37, 1894-1897 wird die aus der Verseifung des Ethylesters erhaltene ethanolisch-alkalische Lösung des Natriumsalzes von ML 3000 mit Phosphorsäure angesäuert und mit einer Mischung aus 3 Teilen Diethylether und 1 Teil Methylenchlorid extrahiert. Der nach Trocknen über Natriumsulfat und nach Abziehen des Lösungsmittelgemisches verbleibende Feststoff wird mit Diisopropylether resuspendiert, abfiltriert und getrocknet. Zur Herstellung von ML 3000 wird auf Arch. Pharm. 321, 159-162 (1988) verwiesen (Diskussion siehe unten).

In Arch. Pharm. Pharm. Med. Chem. 330, 307-312 (1997) werden heterocyclische Strukturanaloga des ML 3000, die nach der Huang-Minlon-Methode aus 2-Oxo-essigestervorstufen gebildet werden, durch Einengen der mit Diethylether von einer kurzen Kieselgelsäule gewonnenen Eluate erhalten.

In Arch. Pharm. 321, 159-162 (1988) werden die Ethylester einiger, zu ML3000 strukturverwandter Säuren in ethanolischer KOH verseift, nach der Verseifung werden die Säuren aus der wässrig-ethanolischen Mutterlauge der Kaliumsalze mittels 6%-iger Phosphorsäure in Freiheit gesetzt und in Diethylether aufgenommen. Nach Volumenreduktion werden die Säuren daraus an neutrales Aluminiumoxid adsorbiert. Nach Etherelution der neutralen Begleitsubstanzen werden die Carbonsäuren vom mineralischen Träger durch Einwirkung wässriger Natriumdihydrogenphosphat-Lösung desorbiert und wiederum in Diethylether aufgenommen. Dieser zweite Diethyletherextrakt wird zur Kristallisation konzentriert, die Kristalle werden abgetrennt und nach Zusatz von Pentan zur Fällung einer zweiten Kristallfraktion das Volumen der etherischen Mutterlauge wieder reduziert.

In der Dissertation Kiefer (Frankfurt, 1992) wird zur Herstellung einer analogen Pyrrolizin-5-yl-essigsäure die entsprechende 2-Oxoessigsäure der Huang-Minlon-Reduktionsmethode unterworfen. Vor der Freisetzung der als Kaliumsalz in der Reaktionsmischung enthaltenen Pyrrolizin-5-yl-essigsäure werden die neutralen bis alkalischen Begleitsubstanzen und Verunreinigungen durch eine Vorextraktion der wässrig-alkalischen Produktphase mit Ethylacetat entfernt. Erst dann wird mittels 6N HCl die Carbonsäure ausgeschieden und in Diethylether aufgenommen. Die Diethyletherauszüge werden mit Wasser gewaschen, getrocknet und das Solvens vollständig entfernt, bis zum Vorliegen eines kristallinen Feststoffes, der anschließend mit kaltem Diethylether gewaschen wird.

In *Tetrahedron 55* (1999), 5145-5156 ist eine alternative Synthese von ML 3000 in acht Stufen beschrieben. Die Schlüsselstufen sind eine thermische, saure Bicyclisierung eines ω-acetylenischen Aminoesters und eine Suzuki Crosskupplungsreaktion zwischen einem Heteroaryltriflat und (4-Chlorphenyl)-boronsäure.

Die nach den bislang bekannten Methoden hergestellten kristallinen Pulverproben des ML 3000 wurden röntgenographisch in Pulverrefraktometern vermessen und die Refraktogramme, die Pulverspektren, miteinander verglichen. Die Substanzproben wurden des weiteren mit der. Differential Scanning Calorimetry-Methode (DSC) oder mit der thermogravimetrischen Methode (TGA)untersucht. Die pulverrefraktometrischen Messungen und die DSC-Messungen zeigen, dass nach Kristallisation aus Diethylether die Substanz zunächst als Ethersolvat in der kristallinen Form von Stäbchen anfällt. Bei Kristallisationen aus Ethylacetat wird analog ein Solvat mit Ethylacetat in Form von Rhomben gebildet. Es hat sich gezeigt, dass diese Solvate instabil sind. Sie zerfallen im Vakuum und/oder bei erhöhten Temperaturen nur unter unvollständiger Abgabe des gebundenen Lösemittels zu weitgehend amorphen Substanzen, die jedoch noch Restlösemittel enthalten und in denen sich nach der Trocknung erhöhte Mengen an Zersetzungssubstanzen nachweisen lassen. Für die Solvate werden mittels DSC Technik charakteristische Desolvations-Temperaturübergänge gefunden.

Rohes ML 3000, das nach dem Hydrazinverfahren als Kaliumsalz anfällt und das dann aus der mit Mineralsäure sauer gestellten Reaktionsmischung ausgeschieden wird, enthält neben den in Wasser schwerlöslichen Kalium-Salzen auch Hydrazin, Nebenprodukte und Zersetzungsprodukte (Decarboxylierungsprodukt sowie Dimer) als Verunreinigung. Dies erforderte zusätzliche Reinigungsoperationen. So mußte z.B. zur Entfernung der Hydrazin-Anteile die rohe kristalline Säure mehrfach mit verdünnten Mineralsäuren gewaschen oder ihre Lösung extrahiert werden, um den Hydrazin-Anteil in der Reinsubstanz in den Bereich unbedenklicher Restmengen abzusenken.

Keines der bislang publizierten Verfahren lieferte ein zur Anwendung am Menschen uneingeschränkt geeignetes Material.

Der vorliegenden Erfindung liegt daher die Aufgabe zugrunde, ein Verfahren zur Herstellung von ML 3000 zur Verfügung zu stellen, bei dem ML 3000 in hoher Ausbeute und reiner, definierter kristalliner Form anfällt.

Überraschenderweise wurde nun gefunden, dass diese Aufgabe gelöst wird, wenn die entsprechende Pyrrolizin-Verbindung mit Oxalylchlorid und Hydrazin umgesetzt und das Reaktionsprodukt einer speziellen Aufarbeitung unterzogen wird. Außerdem wurde gefunden, dass bei der Aufarbeitung ein neues polymorphes ML 3000 (Polymorph A) gebildet wird.

Die vorliegende Erfindung betrifft daher ein Verfahren zur Herstellung der Verbindung der Formel I (ML 3000) 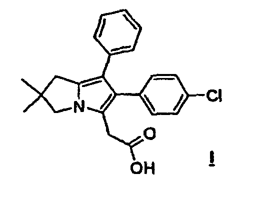 wobei man die Verbindung der Formel III 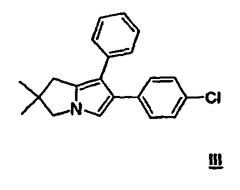 mit Oxalylchlorid umsetzt und das erhaltene Produkt mit Hydrazin und einem Alkalimetallhydroxid in wässriger Phase bei erhöhter Temperatur behandelt, nach beendeter Behandlung durch Zugabe eines mit Wasser nicht oder nur begrenzt mischbaren Ethers ein Drei-Phasen-System erzeugt und die Verbindung der Formel I durch Ansäuern der mittleren Phase gewinnt.

### Kurze Beschreibung der Figuren:

Es zeigen
- Fig. 1: Das DSC-Diagramm eines kristallinen Polymorph (Polymorph A) von ML 3000.
- Fig. 2: Das IR-Spektrum des Polymorph A.
- Fig. 3: Das Röntgenbeugungsspekrum des Polymorph A.
- Fig. 4: Das DSC-Diagramm des Ethylacetat-Solvates von ML 3000 (Polymorph C).
- Fig. 5: Das IR-Spektrum des Polymorph C.
- Fig. 6: Das Röntgenbeugungsdiagramm des Polymorph C.
- Fig. 7: Das DSC-Diagramm des Diethyletherates von ML 3000 (Polymorph E).
- Fig. 8: Das IR-Spektrum des Polymorph E.
- Fig. 9: Das Röntgenbeugungsdiagramm des Polymorph E.

Die Herstellung von 6-(4-Chlorphenyl)-2,2-dimethyl-7-phenyl-2,3-dihydro-1H-pyrrolizin-5-ylessigsäure (ML 3000) unter Verwendung des erfindungsgemäßen Verfahrens läßt sich ausgehend von der Verbindung der Formel IV durch folgende Reaktionsgleichungen darstellen:

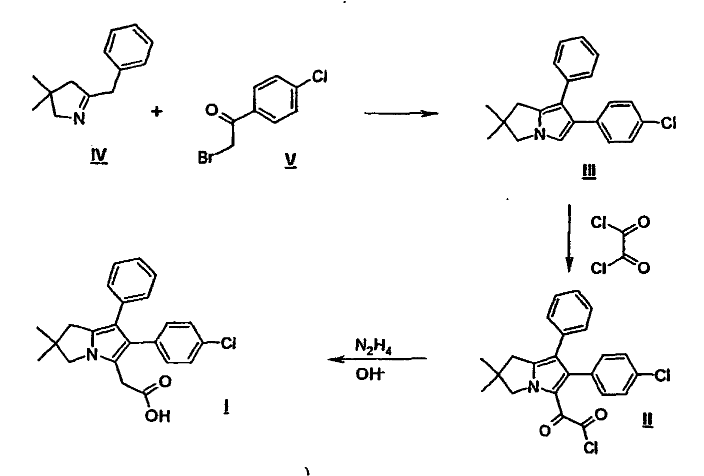

Die Verbindung der Formel IV ist bekannt. Beispielsweise ist sie in Arch. Pharm. 321, 159-162 (1988) beschrieben. Sie läßt sich herstellen durch Umsetzung von Benzylmagnesiumchlorid mit 3,3-Dimethyl-4-chlorbutyronitril, wie J. Med. Chem. 37, 1894-1987 (1994) beschrieben ist. Die Umsetzung erfolgt in einem inerten Lösemittel, wie einem Ether oder einem Kohlenwasserstoff wie Toluol. Die Verbindung der Formel IV wird dann mit einem ω-Brom-4-chloracetophenon V umgesetzt. Die ω-Brom-4-chloracetophenon-Verbindung und ihre Herstellung sind bekannt, sie sind beispielsweise beschrieben in Bull. Soc. Chim. Fr. 21, 69 (1899).

Die Umsetzung erfolgt im allgemeinen in einem polaren organischen Lösemittel. Geeignete polare organische Lösemittel sind insbesondere C₁-C₄-Alkohole, wie Methanol, Ethanol , Isopropanol oder Ether, wie Diethylether, THF, oder Dioxan. Die Reaktionspartner können in äquimolaren Mengen eingesetzt werden. Im allgemeinen verwendet man jedoch das ω-Brom-4-chloracetophenon im Überschuß, beispielsweise in einer Menge von bis zu 40 mol%.

Um den bei der Umsetzung freigesetzten Bromwasserstoff abzufangen, arbeitet man in Anwesenheit einer Base. Vorzugsweise verwendet man eine anorganische Base, insbesondere ein Alkalimetailhydrogencarbonat oder Alkalimetallcarbonat , wobei die entsprechenden Natriumund Kaliumverbindungen besonders bevorzugt sind. Eine anorganische Base kann in Form einer wässrigen Lösung eingesetzt werden. Als besonders bevorzugt hat sich jedoch erwiesen, eine anorganische Base in fester Form zu verwenden. Dies erleichtert die Abtrennung der anorganischen Reaktionsprodukte und verringert das Nebenproduktspektrum. Eine anorganische Base wird im allgemeinen in äquimolaren Mengen eingesetzt, bezogen auf die Menge freigesetzten Bromwasserstoffes. Zweckmäßigerweise verwendet man eine anorganische Base jedoch im Überschuss, beispielsweise bis zu 1,8 Äquivalente. Außerdem hat es sich als zweckmäßig erwiesen, die Umsetzung unter Lichtausschluss vorzunehmen. Die Reaktionstemperatur kann in einem breiten Bereich variiert werden. Vorzugsweise arbeitet man jedoch im Bereich von 0 bis 50 °C.

Die Verbindung der Formel III lässt sich in üblicher Weise gewinnen, indem die gebildeten Salze und das Lösungsmittel entfernt werden. Man erhält auf diese Weise die Verbindung der Formel III in einer Ausbeute von mindestens 40% und mit einer Reinheit von mindestens 97%. Insbesondere beträgt der Gehalt an dem Isomer mit der 4-Chlorphenylgruppe in 5-Position nicht mehr als etwa 1,5% und im allgemeinen etwa 1%.

Die Verbindung der Formel III wird dann mit Oxalylchlorid zur Reaktion gebracht. Im allgemeinen verwendet man ein inertes organisches Lösungsmittel wie einen Ether, insbesondere Diethylether, Methyl-t-butylether, Tetrahydrofuran oder Dioxan, einen Kohlenwasserstoff, wie Toluol oder einen chlorierten Kohlenwasserstoff wie Methylenchlorid. Die Verwendung von Tetrahydrofuran ist bevorzugt.

Die Reaktionstemperatur liegt im allgemeinen im Bereich von -20 bis +30 °C. Um dies zu bewerkstelligen, wird die exotherme Reaktion durch die Zugabegeschwindigkeit des Oxalylchlorids und/oder durch Kühlen des Reaktionsgemisches kontrolliert. Man erhält auf diese Weise die Verbindung der Formel II.

Das Reaktionsgemisch wird dann mit Wasser in Kontakt gebracht, um überschüssiges Oxalylchlorid zu hydrolysieren. Überraschenderweise kommt es dabei nicht zur Hydrolyse der Verbindung der Formel II zur entsprechenden Carbonsäure.

Das Reaktionsgemisch wird dann mit einem Reagens behandelt, das zur Reduktion der Ketocarbonylgruppe in 5-Position unter Bildung der Essigsäuregruppe geeignet ist. Bevorzugt ist zu diesem Zweck die Verwendung von Hydrazin (Wolff-Kishner-Reduktion). Als besonders zweckmäßig hat sich die Huang-Minlon-Variante erwiesen, bei welcher die Umsetzung mit Hydrazin in einem hochsiedenden Alkohol in Anwesenheit eines Alkalimetallhydroxids durchgeführt wird. Zweckmäßigerweise geht man dabei so vor, dass man das für die Umsetzung mit Oxalylchlorid verwendete Lösungsmittel vor oder nach Zugabe des hochsiedenden Alkohols zumindest teilweise entfernt. Anschließend gibt man Hydrazin, insbesondere Hydrazinhydrat zu und erhöht die Reaktionstemperatur auf etwa 70-80 °C, um gegebenenfalls restliches Lösungsmittel abzudestillieren. Im Anschluss daran wird die Reaktionstemperatur auf 120 bis 180 °C, insbesondere 130 bis 160 °C erhöht. Das Alkalimetallhydroxid wird in fester Form oder als konzentrierte wässrige Lauge, jedoch bevorzugt in fester Form zugegeben. Der Zeitpunkt der Zugabe ist nicht kritisch, zweckmäßigerweise gibt man es nach Entfernung des restlichen, für die Umsetzung mit Oxalylchlorid verwendeten Lösungsmittels zu. Vorzugsweise verwendet man Kaliumhydroxid.

Als hochsiedenden Alkohol verwendet man insbesondere einen aliphatischen Mono- oder Dialkohol mit einem Siedepunkt von mindestens 140 °C. Geeignete Alkohole sind Ethylenglykol, Ethylenglykolmonomethylether, etc. und insbesondere Diethylenglykol.

Die Reaktionszeit liegt im allgemeinen im Bereich von 30 bis 300 Minuten.

Die bei der Reaktionstemperatur flüchtigen Bestandteile, bei denen es sich im wesentlichen um Wasser, Hydrazin und gegebenenfalls noch Reste des für die Umsetzung mit Oxalylchlorid verwendeten Lösungsmittels handelt, werden zweckmäßigerweise entfernt, beispielsweise durch Destillation.

Nach beendeter Umsetzung wird das Reaktionsgemisch mit einem Ether (etherisches Solvens) und mit Wasser oder elektrolythaltigem (z.B. NaCl-haltigem) Wasser versetzt. Vorzugsweise verwendet man einen mit Wasser begrenzt mischbaren Ether. Brauchbare Ether sind z.B. Methyl-t-butylether, Tetrahydrofuran und insbesondere Diethylether.

Durch die Zugabe des Ethers kommt es zur Ausbildung eines 3-Phasen-Systems. Die oberste Phase ist eine Etherphase, welche die vorhandenen organischen Verunreinigungen enthält. Die unterste Phase ist eine stark alkalische wässrige Phase, welche die anorganischen Bestandteile enthält. Bei der mittleren Phase handelt es sich um eine ölige Phase, die im wesentlichen aus dem Salz von ML-3000 mit dem bei der Umsetzung verwendeten Alkalimetallhydroxid besteht, das in der Diethylenglykol haltigen alkalischen Wasserphase schwer lösliche ist. Überraschenderweise hat sich gezeigt, dass die mittlere Phase das Salz von ML-3000 in hoher Reinheit enthält.

Die Phasen werden getrennt und die mittlere Phase wird mit einem Gemisch aus Wasser und einem mit Wasser nur begrenzt mischbaren Ether, z.B. Diethylether oder Methyl-t-butylether, versetzt und mit einer anorganischen oder organischen Säure, wie Salzsäure, Schwefelsäure, Phosphorsäure, Essigsäure, Oxalsäure oder Citronensäure bis etwa pH 1 bis 2 angesäuert. Das ML-3000 ist dann in der Etherphase gelöst. Gegebenenfalls kann diese Etherphase in üblicher Weise weiteren Extraktionsschritten mit einer Säure oder Wasser unterworfen werden. Gegebenenfalls kann auch ein weiterer Reinigungsschritt durch Behandeln mit Aktivkohle oder anderen Adsorbentien wie (z.B. Bentonit etc.) angeschlossen werden.

Die Menge an Ether und Wasser, die zur Ausbildung des 3-Phasen-Systems zugegeben wird, ist nicht kritisch. Im allgemeinen wird man soviel Ether und Wasser verwenden, dass sich die Phasen ausbilden und auf einfache Weise trennen lassen. Im allgemeinen verwendet man 5 bis 10 Gewichtsteile Wasser und 3 bis 20 Gewichtsteile Ether pro Gewichtsteil Ausgangsverbindung.

Die Gewinnung des ML-3000 aus der Etherphase kann auf unterschiedliche Weise erfolgen. Beispielsweise kann der Ether verdampft und das ML-3000 durch Kristallisation aus Ethylacetat oder Isopropanol gewonnen werden. Beim Verdampfen des Ethers kristallisiert ein Solvat des Ethers das im Falle des Diethylethers 1 Lösungsmittelmolekül auf 2 Moleküle ML3000 enthält. Aus Ethylacetat wird ein entsprechendes Solvat mit einem Ethylacetatmolekül auf 2 Moleküle ML 3000 erhalten.

Es ist jedoch bevorzugt, zu der Etherphase mindestens einen höher als der Ether siedenden Kohlenwasserstoff zu geben, den Ether gegebenenfalls zumindest teilweise abzudestillieren und das in fester, kristalliner Form abgeschiedene ML-3000 in üblicher Weise, z.B. durch Filtration oder Zentrifugation, von der Mutterlauge zu trennen und durch Trocknen im leichten Vakuum bei geringfügig erhöhten Temperaturen zu gewinnen. Vorzugsweise verwendet man einen Kohlenwasserstoff, der wenigstens 30 °C, insbesondere wenigstens 40 °C, höher siedet als der Ether. Man verwendet soviel Kohlenwasserstoff, dass er, ggf. nach Abdestillieren des Ethers, in etwa 2 bis 15 fachem Überschuss (Volumen) vorliegt.

Bei dem Kohlenwasserstoff kann es sich um einen geradkettigen oder verzweigten aliphatischen Kohlenwasserstoff mit vorzugsweise 6 bis 12 Kohlenstoffatomen handeln. Beispiele sind n-Hexan, n-Heptan, n-Octan, iso-Octan, n-Decan, Cyclohexan, Cycloheptan. Bevorzugt ist n-Heptan oder ein Gemisch isomerer Heptane, sowie Cyclohexan.

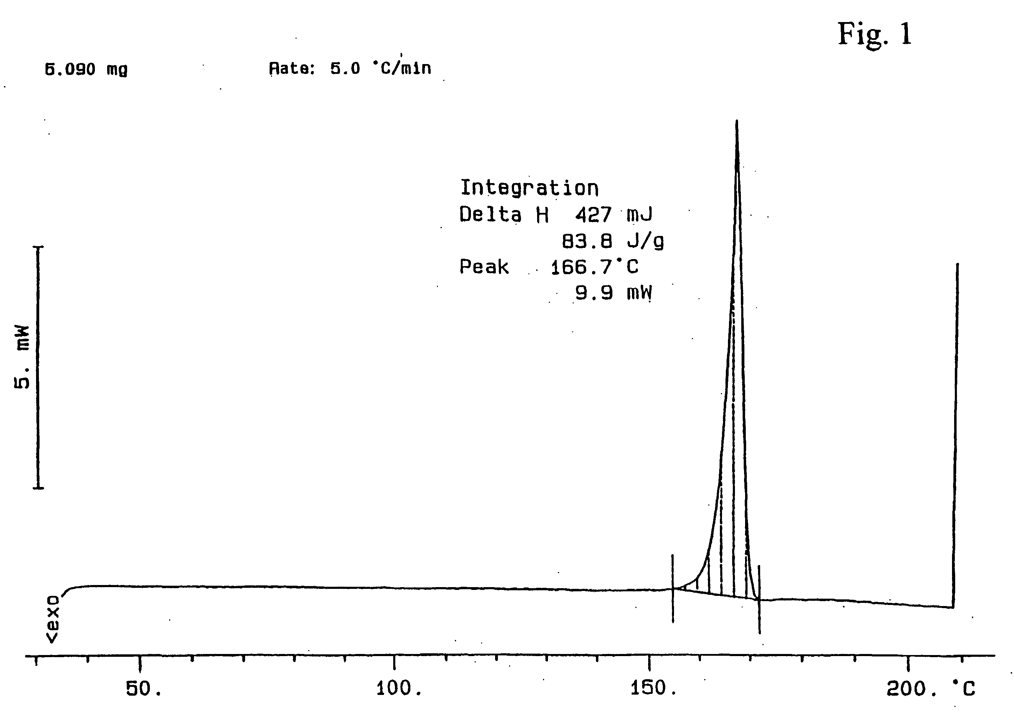

Überraschenderweise hat sich gezeigt, dass man bei Behandlung einer etherischen Lösung von ML 3000, z.B. der erwähnten Etherphase, mit dem Kohlenwasserstoff eine neue, im wesentlichen lösungsmittelfreie Kristallmodifikation des ML 3000, nämlich das Polymorph A, erhält. Polymorph A weist einen einzigen Endotherm im DSC-Diagramm (von 50°C bis 200°C)auf, der im Bereich von 155 bis 170 °C liegt. Das DSC-Diagramm ist in Fig. 1 gezeigt.

Aus amorphem ML 3000 oder anderen Kristallmodifikationen davon (Polymorph C und E, siehe Beispiele 4 und 5) lässt sich Polymorph A durch Behandlung mit dem Kohlenwasserstoff bei erhöhter Temperatur, z.B. bei einer Temperatur im Bereich von 40 bis 110 °C, gewinnen. Zweckmäßigerweise erfolgt die Behandlung mit dem Kohlenwasserstoff durch Ausrühren (Digerieren).

Weiter weist Polymorph A folgende wesentliche Peaks im IR-Spektrum auf (Verreibung mit KBr im Verhältnis 1:3; FT-IR-Spektrometer Spektrum 2000 der Fa. Perkin Eimer; Steuerung des Gerätes mit dem Programm Spektrum 2.00; die Messungen erfolgten in diffuser Reflexion): Wellenzahl (cm⁻¹): 1706; 1601; 1536; 1487; 1463; 1450; 1441; 1413; 1395; 1383; 1369; 1293; 1219; 1177; 1099; 1013; 836; 765; 698.

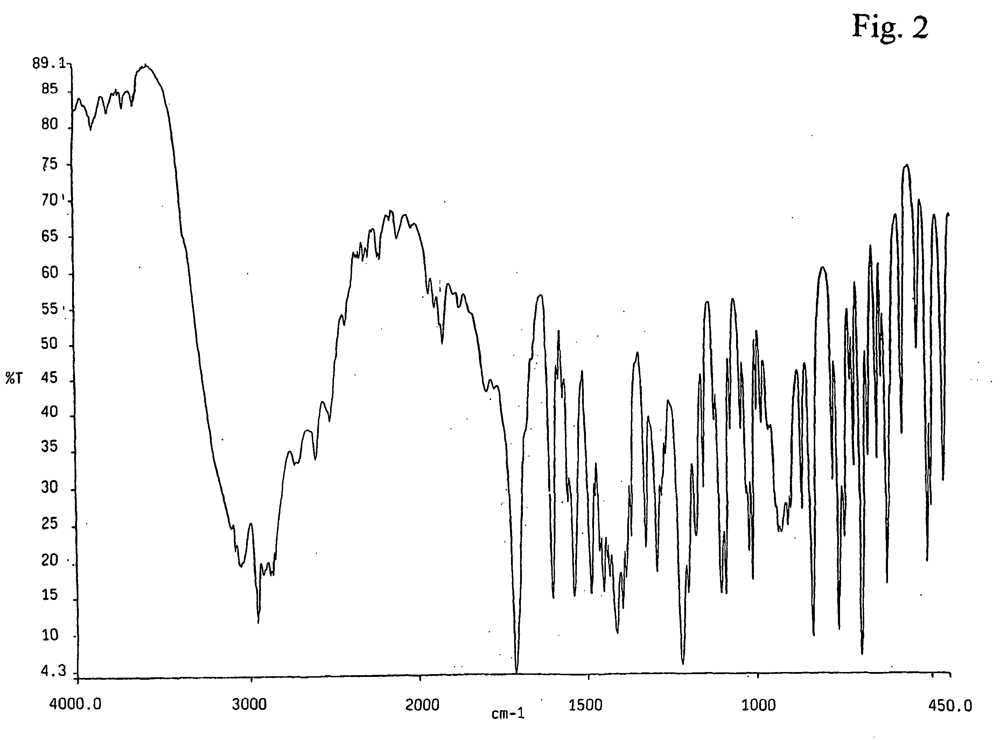

Das IR-Spektrum ist in Fig. 2 gezeigt.

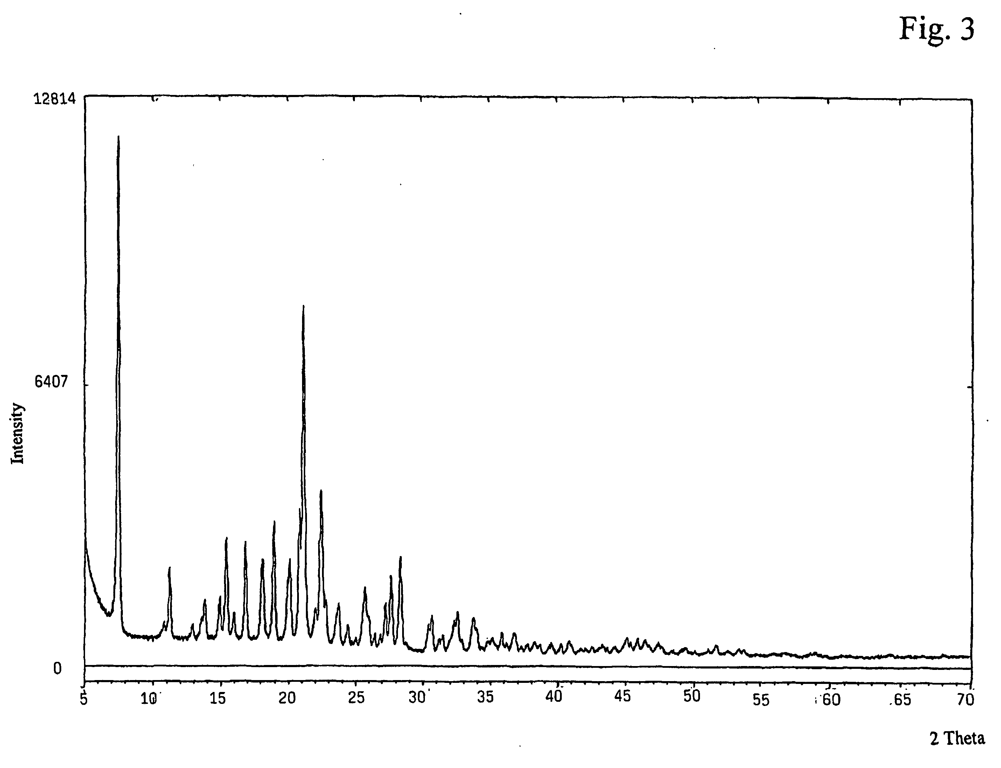

Das Röntgenbeugungsdiagramm (Pulverrefraktogramm) von Polymorph A ist in Fig. 3 gezeigt. Polymorph A besitzt folgende charakteristische d-Werte 11,9; 4,2; 4,0, (2 Θ:7,5;21,2;22,4).

Polymorph A weist eine enge Teilchengrößenverteilung auf, wobei die mittlere Teilchengröße (bestimmt mittels Laser-Beugungsspektren (Laser diffraction spectra) am System der Fa. Helios-Sympatec: Dry disperser RODOS; Focal length 50mm) im Bereich von 30 bis 50 µm liegt. Die Solvate haben dagegen eine breite Teilchengrößenverteilung mit einem hohen Anteil an feinkörnigem Material von etwa 10 µm und einem hohen grobkörnigen Anteil von etwa 1 mm. Die mittlere Teilchengröße liegt im Bereich von etwa 100 bis 150 µm.

Polymorph A besitzt im Vergleich zu den bekannten Solvaten und der amorphen Form von ML 3000 wesentliche Vorteile. Aufgrund der kristallinen Struktur ist Polymorph A bei der Trocknung und der Lagerung stabil. Es sind keine Phasenübergänge und keine Sekundäraggregationen wie bei der amorphen Form zu beobachten. Außerdem ist die Reinheit des Polymorph A höher, weil bei der Gewinnung keine Verunreinigungen in das Kristallgefüge eingeschlossen werden.

Polymorph A weist einen dichten Kristallverbund mit relativ geringer Oberfläche auf Oberflächenphänomene, wie elektrostatische Aufladung, Adhäsion, Adsorption etc., treten im Gegensatz zum amorphen ML 3000 nur in geringem Umfang auf. Die Kristallstruktur bedingt außerdem eine hohe chemische Stabilität, wohingegen das amorphe ML 3000 eine hohe Oberfläche aufweist und daher einer stärkeren oxidativen Zersetzung unterliegt.

Die Solvate sind nicht lagerstabil, weil sie selbst bei Raumtemperatur Lösungsmittel abgeben. Der dabei erfolgende Phasenübergang in die lösungsmittelfreie, amorphe Form verläuft nicht eindeutig. Bei der Alterung der Solvate bleiben Kavitäten im Kristallverbund zurück. Dadurch ist die Substanz einem stärkeren oxidativen Abbau unterworfen. Außerdem erhält man bei der Alterung ein Produkt mit einer breiten Teilchengrößenverteilung, was seine Fließeigenschaften und seine weitere Bearbeitbarkeit ungünstig beeinflusst.

Nach dem erfindungsgemäßen Verfahren erhält man ML-3000 in ausgezeichneter Ausbeute von mindestens 70%, ausgehend von der Verbindung der Formel III. Dies bedeutet eine erhebliche Verbesserung gegenüber dem Stand der Technik. Gemäß Beispiel 5C der WO95/32971 erhält man eine zu ML-3000 analoge Verbindung in lediglich 29%. Überraschenderweise fällt das ML-3000 in hoher Reinheit in lösungsmittelfreier kristalliner Form an. Der Gehalt, bestimmt durch Tetrabutylammoniumhydroxid-Titration, liegt bei 100%. Der Gehalt an Schwermetallen beträgt <10 ppm und die Aschemenge ist 0%. Die Summe an Isomeren und Derivaten des ML-3000 ist <0,2% (bestimmt mittels HPLC), der Gehalt an Restlösemittel liegt unter 0,2% (bestimmt nach der Head Space Gaschromatographie-Methode).

Die erfindungsgemäße Verbindung (Polymorph A) hat sich als potenter Cyclooxygenaseund/oder Lipoxygenasehemmer erwiesen. Sie zeichnet sich durch eine starke analgetische Wirkung und durch eine gleichmäßige Hemmwirkung auf die Enzyme Cyclooxygenase (CO) und Lipoxygenase (LO) (IC₅₀LO/IC₅₀CO ∼ 1) aus. Sie ist daher bei der Behandlung von Erkrankungen brauchbar, die mit einer Veränderung der Arachidonsäuremetabolisierung einhergehen. Insbesondere sind zu nennen Erkrankungen des rheumatischen Formenkreises und die Prävention von allergisch induzierten Erkrankungen. Die erfindungsgemäße Verbindung stellt somit ein wirksames Antiphlogistikum, Analgetikum, Antipyretikum und Antiallergikum dar und ist antibronchokonstriktorisch wirksam und zudem zur Thromboseprophylaxe und zur Prophylaxe des anaphylaktischen und septischen Schocks sowie zur Behandlung dermatologischer Erkrankungen, wie Psoriasis, Urtikaria, akute und chronische Exantheme allergischer und nichtallergischer Genese brauchbar. Außerdem ist sie zur Behandlung von Hypercholesterinämie brauchbar.

Die erfindungsgemäße Verbindung kann entweder als einzelner therapeutischer Wirkstoff oder als Mischung mit anderen therapeutischen Wirkstoffen verabreicht werden. Sie kann als solche verabreicht werden, im Allgemeinen wird sie jedoch in Form eines pharmazeutischen Mittels verabreicht, d.h. als Mischung des Wirkstoffs mit pharmazeutisch akzeptablen Hilfsstoffen, insbesondere Trägerstoffe bzw. Verdünnungsmitteln und/oder Zusatzstoffen. Die Verbindung oder das Mittel können enteral, z.B. oral oder rektal, oder parenteral, z.B. subkutan, intravenös oder intramuskulär verabreicht werden, vorzugsweise werden sie jedoch in oralen Dosierungsformen gegeben.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln vorliegen und können übliche Exzipienzien enthalten, wie Bindemittel (z.B. Sirup, Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talcum, Polyethylenglykol oder Siliciumdioxid), disintegrierende Mittel (z.B. Stärke) oder Netzmittel (z.B. Natriumlaurylsulfat). Orale flüssige Präparate können in Form wässriger oder öliger Suspensionen, Lösungen, Emulsionen, Sirupen, Elixieren oder Sprays usw. vorliegen oder können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren, enthalten. Für die parenterale Verabreichung kann man Lösungen oder Suspensionen mit üblichen pharmazeutischen Trägern einsetzen.

Die Behandlung mit der erfindungsgemäßen Verbindung erfolgt, indem dem zu behandelnden Individuum, vorzugsweise einem Säuger, insbesondere einem Menschen, eine wirksame Menge der Verbindung, in der Regel der pharmazeutischen Praxis entsprechend formuliert, verabreicht wird. Ob eine solche Behandlung angezeigt ist und in welcher Form sie zu erfolgen hat, hängt vom Einzelfall ab und unterliegt einer medizinischen Beurteilung (Diagnose), die vorhandene Anzeichen, Symptome und/oder Fehlfunktionen sowie Risiken, bestimmte Anzeichen, Symptome und/oder Fehlfunktionen zu entwickeln, und weitere Faktoren miteinbezieht.

Die Behandlung erfolgt in der Regel durch einmalige oder mehrmalige tägliche Verabfolgung gegebenenfalls zusammen oder im Wechsel mit anderen Wirkstoffen oder wirkstoffhaltigen Präparaten, so dass einem zu behandelnden Individuum eine Tagesdosis von etwa 0,1 mg bis etwa 1000 mg und insbesondere 0,5 mg bis etwa 100 mg pro kg Körpergewicht zugeführt wird.

Die nachfolgenden Beispiele erläutern die Erfindung, ohne sie zu begrenzen.

Die im Rahmen der vorliegenden Erfindung angegebenen DSC-Diagramme und Röntgenbeugungsspektren (Pulverrefraktogramme) wurden wie folgt erhalten:

Die DSC-Analysen wurden mit dem TA 4000-System der Fa. Mettler durchgeführt (Meßzelle DSC-20; Prozessor T11; Auswertung mit Programm TA-72). Die Heizrate war 5 °C/min, im Schmelzbereich 2 °C/min.

Die Pulverrefraktogramme wurden mit einem Pulverdiffraktometer STOE Powder Diffraction System der Fa. Stoe, Darmstadt, unter Verwendung monochromatischer CuKα1-Strahlung bestimmt.

### Beispiel 1

### 6-(4-Chlorphenyl)-2,2-dimethyl-7-phenyl-2,3-dihydro-1H-pyrrolizin

In einem 250 L Reaktor werden, nach dreimaligem Evakuieren und N2-Einleiten, nacheinander 4,64 kg (190,9 Mol) Magnesium und 18,8 kg Diethylether vorgelegt. Der Ether wird zum Refluxieren gebracht. Bei ausgeschaltetem Rührer werden 0,03 kg Jod und 0,5 kg (4 Mol) Benzylchlorid zugegeben, worauf die Reaktion des Magnesium mit dem Halogenid sofort anspringt (Entfärbung und Trübung). Bei eingeschaltetem Rührer wird eine Lösung von 23,5 kg (185,6 Mol) Benzylchlorid in 37,8 kg Diethylether aus einem Zulaufgefäß während 2 h zugegeben, wobei die schwarz-graue Mischung stark refluxiert. Nach beendeter Zugabe wird die Grignardlösung noch 2 h am Rückfluss gehalten. Anschließend wird bei Rückflusstemperatur aus dem Zulaufgefäß eine Lösung von 17,7 kg (134,6 Mol) destilliertem 4-Chlor-3,3-dimethylbutyronitril in 48,5 kg Diethylether während 1,5 h zugegeben. Die Reaktionsmischung wird noch 2 h zum Rückfluss erhitzt. Anschließend wird aus der hellgrauen Suspension der Diethylether bei Normaldruck abdestilliert. Es werden 54-59 kg Destillat abgenommen (Zeitbedarf 2 h), so dass das Reaktionsgemisch noch rührbar bleibt.

Zum Rückstand werden 106,3 kg Toluol gegeben. Die Innentemperatur beträgt 43 °C. Anschließend wird bis zum Erreichen einer Innentemperatur von 85-90 °C ein Ether/Toluol-Gemisch abdestilliert (ca. 36-40 kg Destillat).

Der Rückstand wird zu einer dicken, aber noch rührbaren Suspension ohne Kruste. Diese Suspension wird in einen Reaktor transferiert, in dem zuvor 76,7 kg Eis und 38,5 kg 32%-ige Salzsäure vorgelegt worden sind. Beim Eintrag steigt die Innentemperatur der Phasen von 0 auf 23 °C an. Der pH-Wert der Wasserphase sollte zwischen 0,5 und 1,5 liegen (pH = 1,0). Nach Erwärmung des Reaktors auf eine Innentemperatur von 40-45 °C werden die Phasen 1,75-2 h kräftig untereinander gerührt. Danach läßt man zur Phasentrennung bei dieser Temperatur und bei ausgeschalteter Rührung 10-15 min stehen. Die das Produkt enthaltende Wasserphase wird abgetrennt (147 kg).

In einer Extraktionsapparatur wird die Wasserphase auf -8 bis 0 °C abgekühlt und dann mit 33,2 kg 24%-igem Ammoniak alkalisiert, wobei die Zulaufgeschwindigkeit des Ammoniaks so geregelt wird, dass die Innentemperatur ein Maximum von 5 °C nicht überschreitet. Der pH-Wert liegt bei 10,5-11.

Die alkalisierte Wasserphase wird mit 106,3 kg Diethylether 30-40 min bei 10-25 °C gut durchgerührt und dann 25-30 min zur Phasentrennung stehen gelassen. Die klare, schwach gelbe Wasserphase (170 kg) wird abgetrennt und verworfen. Die klare, gelblich grüne Etherphase wird unter Vakuum (0,7-0,8 mbar) vollständig eingeengt, wobei 95 kg Etherdestillat erhalten werden (1,40 h). Als Destillationsrückstand erhält man 20,6 kg hellgrünes Öl, das 86,7% 2-Benzyl-4,4-dimethyl-1-pyrrolin enthält. 20,6 kg des Rückstandes (86,7%-ig), entsprechend 17,9 kg (95,5 Mol) 2-Benzyl-4,4-dimethyl-1-pyrrolin, 29,7 kg (127,2 Mol, 1,33 Äquiv.) ω-Brom-4-chloracetophenon und 226,6 kg Methanol werden in einem Reaktor (500 L) vorgelegt. Nach Zugabe von 12,7 kg (151,2 Mol, 1,58 Äquiv.) Natriumhydrogencarbonat wird unter Lichtausschluß bei 17-24 °C unter Bildung einer beigen Suspension gerührt. Die Reaktion wird weitergeführt, bis der Restgehalt an Pyrrolinverbindung in der Mischung <5% beträgt. Nach 17 h wird eine Probe gezogen und mittels Gaschromatographie auf Gehalt an Pyrrolinverbindung geprüft. Die Analyse ergab einen Gehalt von 2%. Danach wird die Suspension bei einer Innentemperatur von 18-22 °C zentrifugiert und der durch Zentrifugation erhaltene Feststoff wird mit 14,4 kg Methanol in 2 Portionen ausgewaschen. Das noch feuchte, schwach gelbe Produkt wiegt 25,8 kg.

Das noch feuchte Rohprodukt (25,8 kg) wird in 150 kg Wasser suspendiert, dann innerhalb 15 min auf eine Innentemperatur von 50-60 °C erwärmt und 40 min bei dieser Temperatur gerührt. Die auf 40 °C abgekühlte Suspension (40 min) wird zentrifugiert und der durch Zentrifugieren erhaltene hellgelbe, kristalline Feststoff wird mit 27 kg Wasser in 2 Portionen nachgewaschen. Das Produkt wird im Vakuum bei 50-60 °C während 12-24 h getrocknet. Man erhält 18,6 kg 6-(4-Chlorphenyl)-2,2-dimethyl-7-phenyl-2,3-dihydro-1H-pyrrolizin, mit einem Gehalt an 0,33% Asche und einem Isomerengehalt an (5-(4-Chlorphenyl)-2,2-dimethyl-7-phenyl-2,3-dihydro-1H-pyrrolizin) von 1,0%.

### Beispiel 2

### 6-(4-Chlorphenyl)-2,2-dimethyl-7-phenyl-2,3-dihydro-1H-pyrrolizin-5-yl-essigsäure (ML-3000)

In einem 250 L Reaktor werden, nach dreimaligem Evakuieren und N2-Einleiten, 11,5 kg (35,7 Mol) 6-(4-Chlorphenyl)-2,2-dimethyl-7-phenyl-2,3-dihydro-1H-pyrrolizin in 60 kg Tetrahydrofuran (THF) vorgelegt. Unter 0,5 bar Stickstoffzufluss (N2) wird die gelbgefärbte Lösung auf 10-15 °C abgekühlt. Anschließend werden unter N2 6,8 kg (54,7 Mol) Oxalylchlorid aus einem Zulaufgefäß über 35 min so zudosiert, dass die Innentemperatur 20 °C nicht überschreitet.

Nach beendeter Zugabe wird die nun dunkelgrüne, dünne Suspension 20-30 min bei einer Innentemperatur von 18-25 °C nachgerührt.

In einem 500 L Reaktor, werden 18 kg Eis in Schuppen vorgelegt. Zu diesem Eis wird die 25 °C warme Suspension über 5 min so zudosiert, dass die Innentemperatur des Gemisches 20 °C nicht überschreitet.

Das Reaktionsgemisch wird noch 10-20 min bei einer Innentemperatur von 25-35 °C gerührt. Die noch immer grüne Lösung wird bei 25-35 °C mit 62,2 kg Diethylenglykol verdünnt. Dann werden aus einem Zulaufgefäß unter Kühlung 14,9 kg (298 Mol) Hydrazinhydrat während 10-15 min zugegeben. Die Innentemperatur steigt auf maximal 40-45 °C an. Durch schrittweises Erhöhen der Temperatur während 1,5 h wird die inzwischen beige gefärbte Suspension auf eine Innentemperatur von 70-75 °C erwärmt, wobei THF abdestilliert wird. Bis zum Erreichen einer Innentemperatur von 75 °C werden 45,4 kg THF-Destillat gesammelt.

Das Reaktionsgemisch wird auf 50-55 °C abgekühlt und in 8 bis 10 Portionen über 45 min verteilt mit insgesamt 26,4 kg Kaliumhydroxid in Schuppen (KOH) versetzt, wobei die Innentemperatur bereits bei den ersten 5 kg KOH auf 65-70 °C ansteigt und die anfangs dicke Suspension sich gelb verfärbt, dünnflüssiger wird und kurzfristig leichter Rückfluss auftritt.

Diese Suspension wird nun mit einem Temperaturanstieg von 15 °C/h auf 90 °C erwärmt, wobei ab 85 °C ein leichtes Schäumen einsetzt und die Suspension eindickt. Mit einer Temperatursteigerung von 2 °C/h wird die Innentemperatur nun weiter auf 102 °C angehoben und gleichzeitig bei erhöhter Rührerdrehzahl durch das Tauchrohr Stickstoff durch die Reaktionsmischung geblasen. Durch starkes Aufschäumen und zusätzliche Gasentwicklung nimmt das Volumen des Reaktorinhaltes auf das Doppelte zu. Bei Bedarf wird die Reaktionstemperatur durch Kühlung abgesenkt. Bei einer Innentemperatur von 100-105 °C beginnt der Schaum zusammenzufallen und eine rotbraune dünne Suspension entsteht, die nun mit einer Heizgeschwindigkeit von 15 °C/h weiter auf eine Innentemperatur von 140-145 °C aufgeheizt wird. Bei übermäßigem Schäumen wird die Reaktionstemperatur durch Kühlung kurzzeitig abgesenkt. Gleichzeitig werden mehrere wässrige Destillate von insgesamt 44 kg gesammelt.

Der Ansatz wird 2 h - 2,5 h bei 120-145 °C gehalten. Danach wird die Reaktortemperatur auf 30-40 °C abgekühlt und es werden
74,7 kg Wasser und 56,7 kg Diethylether zugegeben. Die Reaktionsmischung wird 10-15 min bei einer Innentemperatur von 30-33 °C durchgerührt, dann läßt man die Phasen absitzen. Das entstehende Dreiphasensystem wird getrennt. Die unterste stark alkalische wässrige Phase, die 154,9 kg wiegt, ist farblos und nur schwach getrübt. Sie wird als Abwasser entsorgt. Die gelbgefärbte, trübe Zwischenphase von öliger Konsistenz wiegt 29,6 kg und enthält die Hauptmenge an Produkt als Kaliumsalz. Die oberste, klare, gelbgefärbte etherische Phase wird bei einer Innentemperatur von 30 °C in einer Extraktionsapparatur mit 10 kg Wasser 10 min kräftig gerührt. 10 min nach Abstellen der Rührung wird die Wasserphase abgetrennt. Die Zwischenphase (29,6 kg) und der wässrige Extrakt der Etherphase (10,9 kg) werden in einer Extraktionsapparatur mit 126,2 kg Diethylether und 59,7 kg Wasser versetzt und das Gemisch wird auf eine Innentemperatur von 0-5 °C gekühlt.

Über ein Zulaufgefäß wird nun ein Gemisch aus 6,0 kg
32,5%-iger Salzsäure und 6,0 kg Wasser während 15 min so zudosiert, dass eine maximale Innentemperatur von 10 °C nicht überschritten und ein pH-Wert von 1-2 erreicht wird. Ist dieser pH-Wert nicht erreicht, werden weitere 0,2 kg
32,5%-iger Salzsäure im Gemisch mit 0,2 kg Wasser zugegeben. Nach Erreichen dieses pH-Wertes werden die Phasen noch 5-10 min gut durchgerührt, und dann zur Phasentrennung bei abgeschalteter Rührung 10-20 min stehen gelassen.

Die HCl-saure Wasserphase wird abgelassen. Die Etherphase wird nochmals über das Zulaufgefäß mit einer Mischung aus 9,5 kg Salzsäure und 19 kg Wasser versetzt und bei einer 10 °C nicht überschreitenden Innentemperatur 5-10 min gut durchgerührt. Die Phasen werden getrennt und die HCl-Behandlung wird gewünschtenfalls bis zu 3 mal wiederholt.

Die Etherphase wird danach mit 30 kg demineralisiertem Wasser versetzt, während 10-20 min gut durchgerührt und auf 15-20 °C erwärmt. Man trennt die Phasen und wiederholt die Extraktion.

Die von Säurespuren freigewaschene Etherphase wird mit 6,5 kg wasserfreiem Magnesiumsulfat und 0,4 kg Aktivkohle (Acticarbon 2S), die in 1 kg Diethylether angeschlämmt werden, versetzt und 30-45 min bei 18 °C gerührt. Die Suspension wird über ein mit 0,5 kg Filtrierhilfsmittel (Cell Flock) belegtes Druckfilter in eine Destillationsapparatur klarfiltriert. Filter und Apparatur werden mit 8 kg Diethylether nachgespült.

Zur Etherphase werden 95,6 kg n-Heptan gegeben und bei einer Innentemperatur von 15-20 °C wird der Ether unter Vakuum abdestilliert. Die nach Abdestillieren des Ethers entstandene Kristallsuspension wird auf eine Innentemperatur von 13-18 °C gekühlt und 0,5-1,5 h bei dieser Temperatur gerührt, anschließend werden die Kristalle abzentrifugiert. Das gewonnene feuchte Produkt wird mit 23,0 kg n-Heptan in 2 Portionen gewaschen. Das feuchte Produkt wird bei 50-60 °C über Nacht im Vakuumtrockenschrank getrocknet und gewünschtenfalls vermahlen. Man erhält 10,5 kg (77,2%) ML-3000.

### Beschreibung des Produktes:

Das Produkt hat eine schwach gelbliche bis elfenbein-farbene Färbung. Die Lösung in Tetrahydrofuran ist farblos (Y7) und klar. Der Schmelzpunkt, bestimmt nach der DSC-Methode (unter anderen Bedingungen als oben angegeben) liegt bei 157 °C. Eine zweite Bestimmung des Schmelzpunktes unter den oben angegebenen Bedingungen hat das in Fig.1 gezeigte DSC-Diagramm ergeben.

Das IR-Spektrum und das Pulverrefraktogramm ist in den Figuren 2 und 3 gezeigt. Die Netzebenenabstände (d-Werte) sind wie folgt (berücksichtigt wurden alle Peaks bis 2 Θ = 34):

| 2 Θ | d-Wert | rel. Intensität (%) |
|---|---|---|
| 7.5 | 11.9 | 100.0 |
| 10.8 | 8.2 | 8.3 |
| 11.2 | 7.9 | 18.6 |
| 13.8 | 6.4 | 12.6 |
| 14.9 | 5.9 | 12.7 |
| 15.4 | 5.8 | 24.2 |
| 15.9 | 5.6 | 10.0 |
| 16.8 | 5.3 | 23.4 |
| 18.1 | 5.0 | 20.1 |
| 18.15 | 4.9 | 20.1 |
| 19.0 | 4.7 | 27.4 |
| 19.9 | 4.5 | 16.8 |
| 20.1 | 4.4 | 20.2 |
| 20.8 | 4.3 | 30.0 |
| 21.2 | 4.2 | 68.5 |
| 22.0 | 4.05 | 10.8 |
| 22.4 | 4.0 | 33.3 |
| 22.8 | 3.9 | 12.5 |
| 23.7 | 3.7 | 11.9 |
| 24.4 | 3.6 | 7.7 |
| 25.0 | 3.55 | 5.4 |
| 25.7 | 3.5 | 14.8 |
| 26.4 | 3.4 | 6.0 |
| 26.9 | 3.3 | 6.0 |
| 27.2 | 3.25 | 11.8 |
| 27.7 | 3.2 | 17.0 |
| 28.4 | 3.1 | 20.2 |
| 30.4 | 2.95 | 7.8 |
| 30.7 | 2.9 | 9.4 |
| 31.2 | 2.85 | 5.1 |
| 31.5 | 2.8 | 5.8 |
| 32.3 | 2.75 | 8.6 |
| 32.5 | 2.7 | 10.2 |
| 33.7 | 2.65 | 9.0 |
| 33.9 | 2.6 | 7.0 |

Der Gehalt, bestimmt durch Tetrabutylammoniumhydroxid-Titration liegt bei 100,9%.

| | |
|---|---|
| Gehalt an Schwermetallen | <10 ppm. |
| Aschegehalt | 0% |
| Gehalt an Restlösemitteln (bestimmt mit GC) | 0,11% Diethylether und 0,04% Heptan |
| Hydrazingehalt | <0,3 ppm. |

### Beispiel 3

### Herstellung von Polymorph A der 6-(4-Chlorphenyl)-2,2-dimethyl-7-phenyl-2,3-dihydro-1Hpyrrolizin-5-yl-essigsäure durch Kristallisation aus Diethylether / Cyclohexan

Bei erhöhter Temperatur (40-50 °C) wird die wasserfeuchte rohe 6-(4-Chlorphenyl)-2,2-dimethyl-7-phenyl-2,3-dihydro-1H-pyrrolizin-5-yl-essigsäure (500 g) vollständig in Diethylether (13 L) unter Rühren gelöst, über Aluminiumoxid Al2O3 filtriert und aus dem Filtrat das Solvens entfernt. Die als Rückstand erhaltene rohe Kristallfraktion wird in Cyclohexan (3,6 L) suspendiert und in der Hitze digeriert. Nach Abkühlen auf Raumtemperatur werden die Kristalle abfiltriert, mit kaltem Cyclohexan, dann mit kaltem Methanol gewaschen und anschließend mehrere Stunden bei 50 - 60 °C getrocknet. 470g (73% bez. auf eingesetzte Vorstufe) Polymorph A der 6-(4-Chlorphenyl)-2,2-dimethyl-7-phenyl-2,3-dihydro-1H-pyrrolizin-5-ylessigsäure in 99.69% Reinheit werden erhalten.

### Beispiel 4

### Herstellung von 6-(4-Chtorphenyl)-2,2-dimethyl-7-phenyl-2,3-dihydro-1H-pyrrolizin-5-ylessigsäure · ½ Ethylacetat (Polymorph C).

Die in Ethylacetat (115 L) bei erhöhter Temperatur (40 -50 °C) suspendierte rohe 6-(4-Chlorphenyl)-2,2-dimethyl-7-phenyl-2,3-dihydro-1H-pyrrolizin-5-yl-essigsäure (33 Kg) wird unter Rückflusskochen in Lösung gebracht. Die nun klare Lösung wird durch einen beheizten Filtertrichter filtriert und dann langsam unter Rühren innerhalb 2 - 2.5 h mit einer Kühlrate von 0.5 °C / min. auf 15 - 20 °C abgekühlt. Man läßt die Kristalle bei ausgeschalteter Rührung während 20 min. bei dieser Temperatur absitzen, zentrifugiert und wäscht mit kaltem Ethylacetat (33 L).
Der Gehalt an Ethylacetat wurde mittels 1H-NMR-(CDCl3) aus den nach Abpressen und Lufttrocknen frisch gewonnenen Kristallfraktionen bestimmt. Der erwartete theoretische Wert von 10.38 Gewichtsproz. (%) für das 2:1 Solvat (Hemisolvat) wurde annähernd erreicht. Aus dem Integral über der Resonanzlinie für die Acetyl-Methylgruppe des Ethylacetates (d = 2.04, 3H) zum Integral über der Resonanzlinie der Methylengruppe des ML 3000 bei d (ppm) = 2.85, (2 H) ergibt sich das für das Hemisolvat zu erwartende numerische Verhältnis von 3:4.

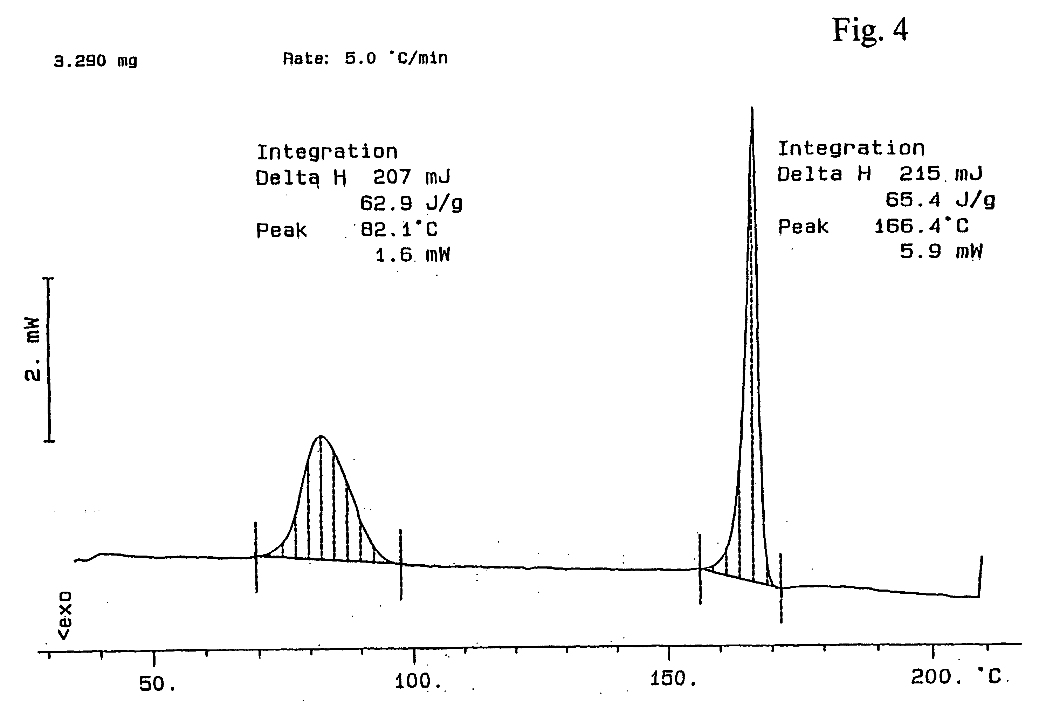

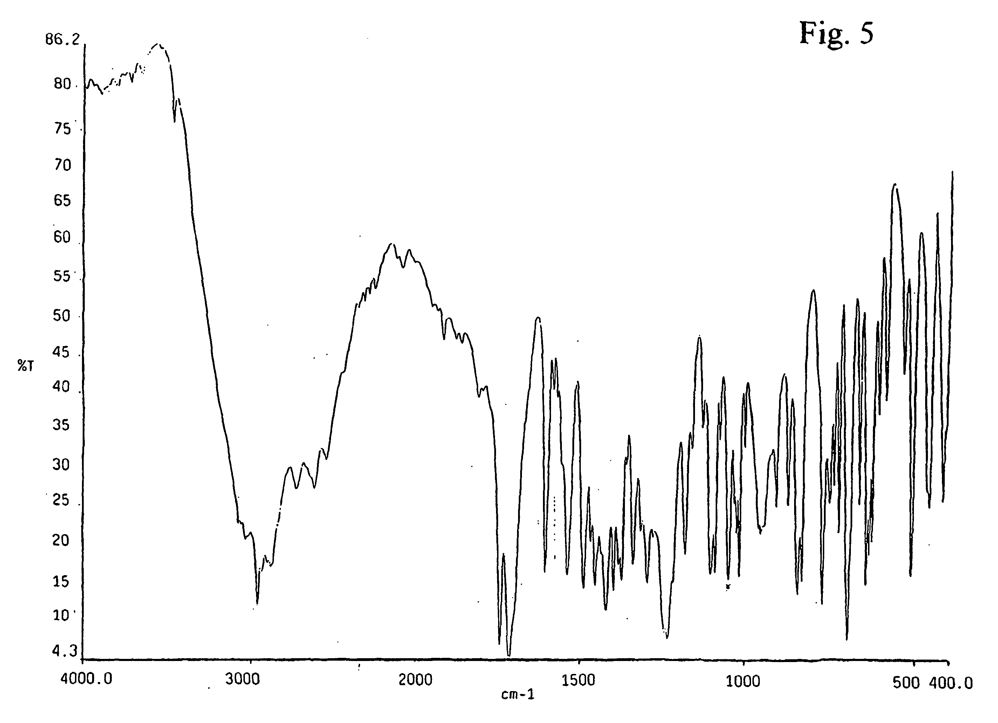

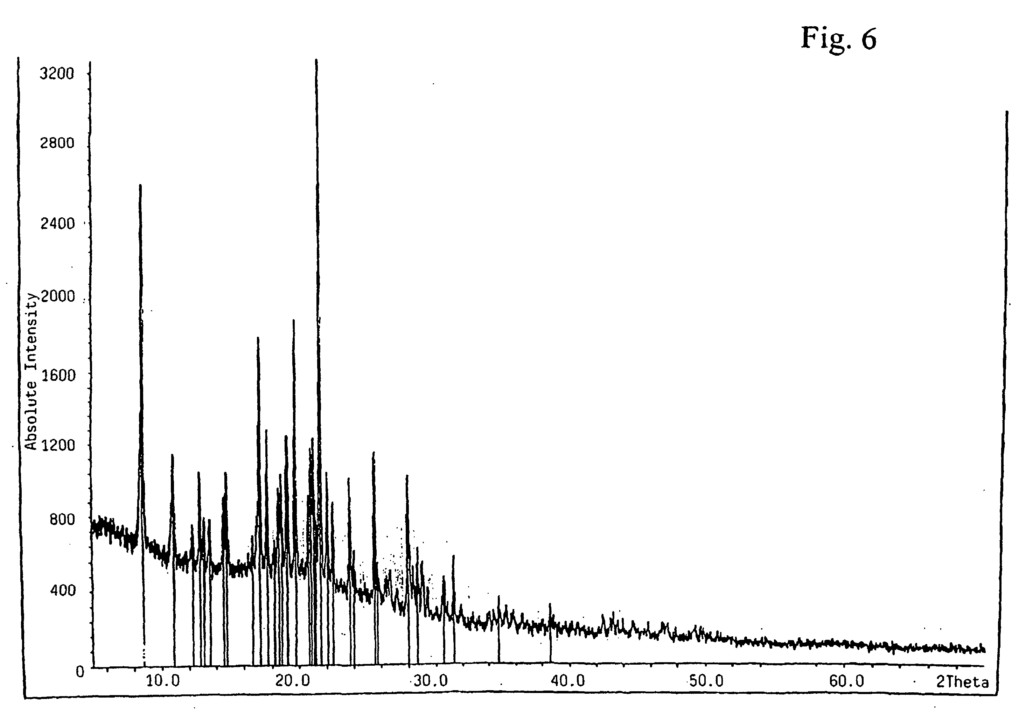

Das DSC-Diagramm, das IR-Spektrum und das Pulverrefraktogramm sind in den Fig. 4, 5 und 6 gezeigt.

### Beispiel 5

### Herstellung von 6-(4-Chlorphenyl)-2,2-dimethyl-7-phenyl-2,3-dihydro-1H-pyrrolizin-5-ylessigsäure · ½ Diethylether (Polymorph E)

6-(4-Chlorphenyl)-2,2-dimethyl-7-phenyl-2,3-dihydro-1H-pyrrolizin-5-yl-essigsäure (2 g) werden in Diethylether (36 mL) unter Rückflusskochen vollständig in Lösung gebracht. Unter Lichtausschluß wurde über 7 Stunden hinweg langsam auf Raumtemperatur abgekühlt. Beim weiteren Absenken der Temperatur auf 0°C erfolgt ab 15 °C die Bildung von Kristallen. Zum Kristallwachstum wurde für 2 Tage bei 0°C gelagert, danach die Mutterlauge von den Kristallen dekantiert.

Der Gehalt an Diethylether wurde mittels 1H-NMR-(CDCl₃) aus den nach dem Dekantieren und Lufttrocknen frisch gewonnenen Kristallen bestimmt. Der erwartete theoretische Wert von 8,87 Gewichtsproz. (%) für das 2:1 Solvat (Hemisolvat) wurde näherungsweise erreicht. Aus dem Integral über dem Triplett für die Ethyl-Methylgruppen des Diethylethers (δ = 1.21, 6H) zum Integral über der Resonanzlinie der geminalen Dimethylgruppe des ML 3000 (δ = 1.29, 6 H) ergibt sich das für das Hemisolvat erwartete numerische Verhältnis von 1:2.

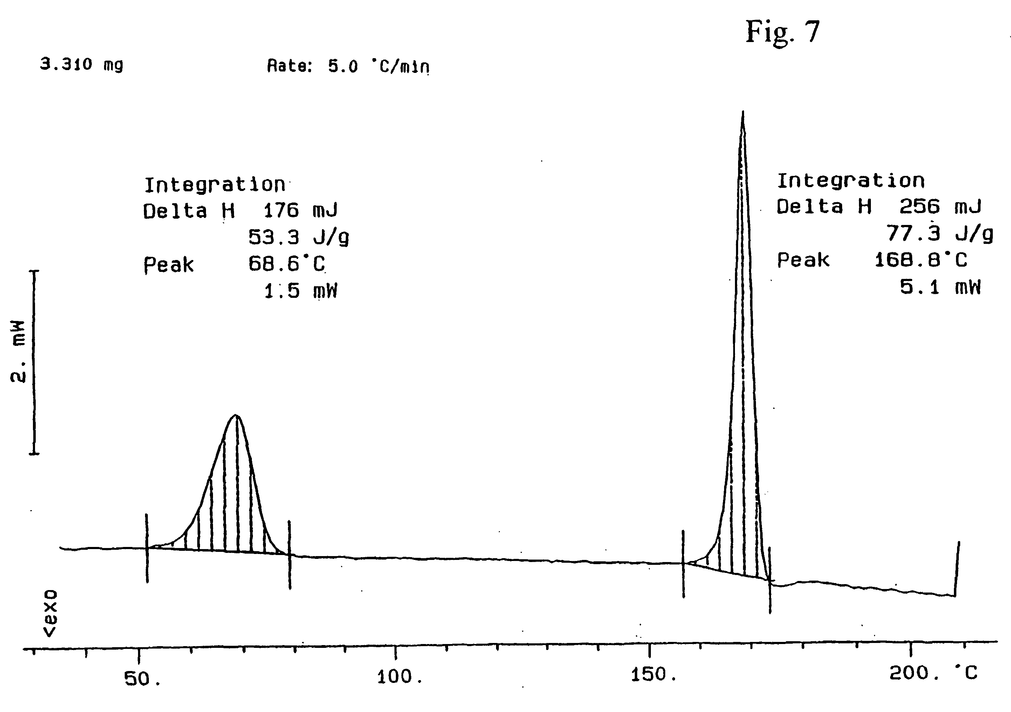

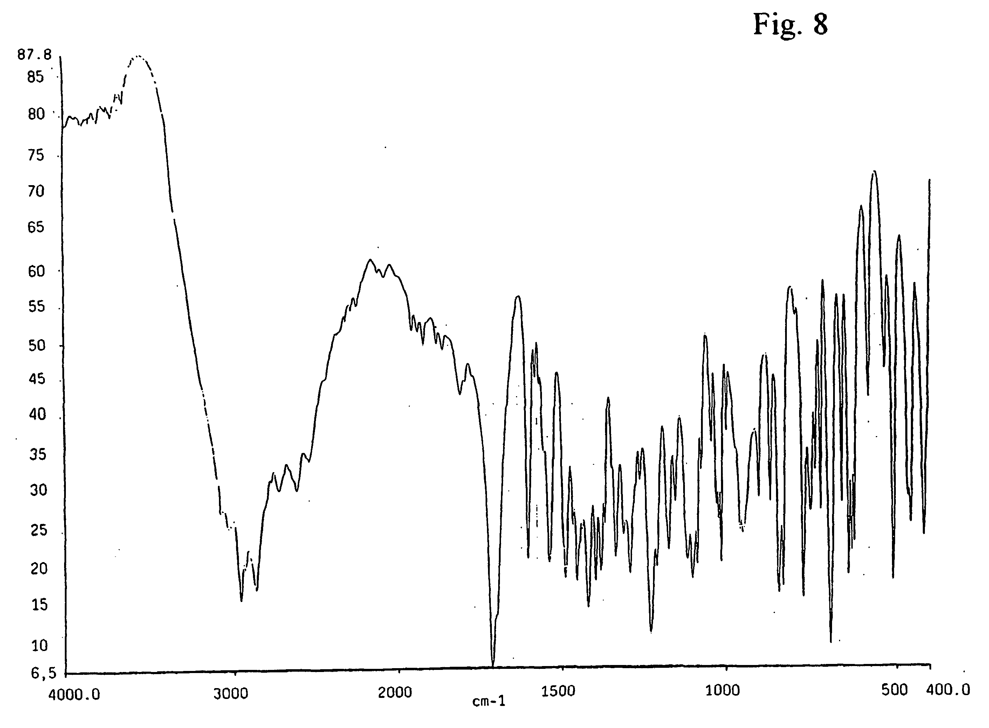

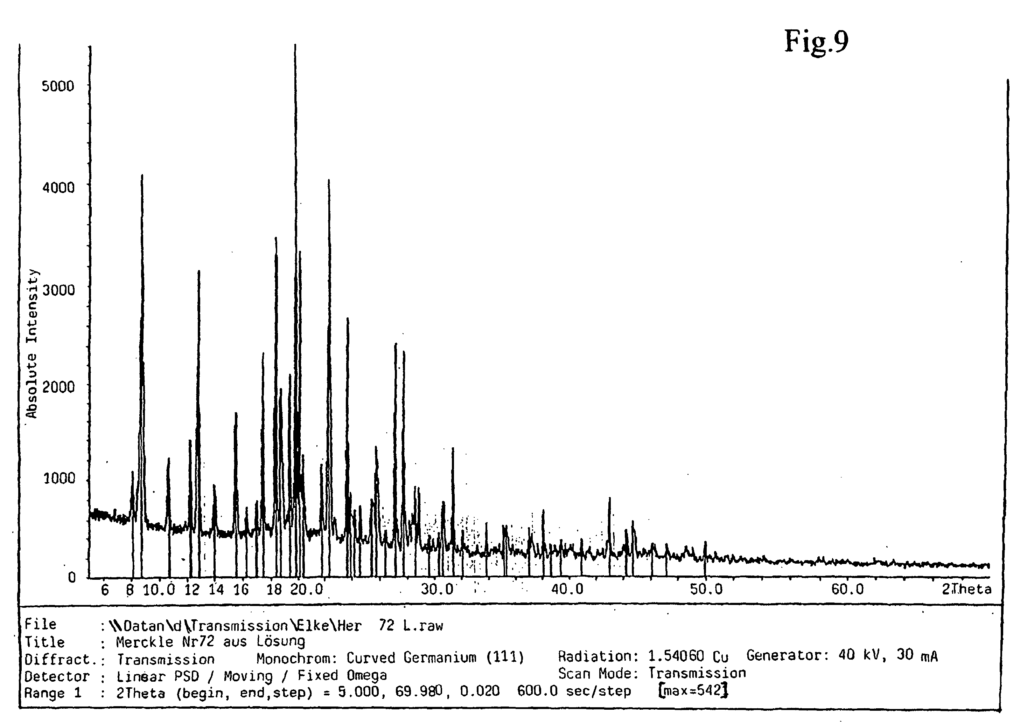

Das DSC-Diagramm, IR-Spektrum und das Pulverrefraktogramm sind in den Fig. 7, 8 und 9 gezeigt.

### Beispiel 6

### Herstellung von Einkristallen des Polymorph E

6-(4-Chlorphenyl)-2,2-dimethyl-7-phenyl-2,3-dihydro-1H-pyrrolizin-5-yl-essigsäure (250 mg) werden unter Zusatz von 1,4-Dichlorbenzol (0,5mL) in Diethylether (5 mL) aufgelöst. Unter Lichtausschluß wurde eine Stunde bei 4°C, danach auf -25 °C gekühlt. Die Substanz kristallisiert im Verlauf eines Tages in Form großer, gut ausgebildeter farbloser Stäbchen.

### Beispiel 7

### Herstellung von Polymorph A der 6-(4-Chlorphenyl)-2,2-dimethyl-7-phenyl-2,3-dihydro-1Hpyrrolizin-5-yl-essigsäure durch Kristallisation aus Methyl-tert.-butyl-ether (MTB) / n-Heptan

In einem 100 ml 2-Hals-Kolben, der ausgestattet ist mit Rückflusskühler und Magnetrührstäbchen und zum Schutz vor Lichteinfall mit Aluminiumfolie umwickelt ist, wird ML 3000 (1 g) in Methyl-tert.-butylether (14 mL) suspendiert und durch Erhitzen bis zur Siedetemperatur unter Argonatmosphäre (1.2 atm) vollständig in Lösung gebracht. Nach 15 minütigem Rühren (200 rpm) unter Rückfluss wird zu der klaren Lösung n-Heptan gegeben bis zur beginnenden Trübung (30 mL) dann langsam in der Wärme weiteres n-Heptan (40 mL), bis zur Bildung einer Suspension (65°C), die man anschließend unter Rühren auf Raumtemperatur abkühlen läßt. Nach 3 Stunden wird die Suspension weitere 15 Stunden im Kühlschrank bei 4°C gelagert. Die Kristalle werden mit mildem Vakuum (500 mbar) von der Mutterlauge über einen G4-Glassinterfilter abgesaugt. Der Kristallkuchen wird in n-Heptan (10 mL) wiederholt (5 mal) resuspendiert und trocken gesaugt (1 min.) und dann ohne weiteres Trocknen in einem verschlossenen Glas aufbewahrt. Die Ausbeute an Kristallen beträgt 81%, sie sind von rein weißer Farbe. Die Kristalle zeigen das Pulverrefraktogramm des reinen Polymorphen A.

## Patentansprüche

1. Verfahren zur Herstellung der Verbindung der Formel I 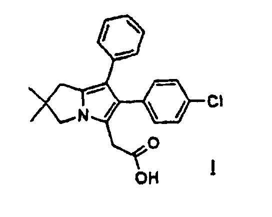 wobei man die Verbindung der Formel III 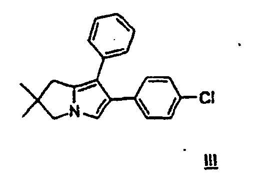 mit Oxalylchlorid umsetzt und das erhaltene Produkt mit Hydrazin und einem Alkalimetallhydroxid in wässriger Phase bei 120 bis 180 °C in Anwesenheit eines aliphatischen Mono- oder Dialkohols mit einem Siedepunkt von mindestens 140 °C behandelt, nach beendeter Behandlung durch Zugabe eines Ethers ein 3-Phasen-System erzeugt und die Verbindung der Formel I durch Ansäuern der mittleren Phase gewinnt.

2. Verfahren nach Anspruch 1, wobei man als Ether, Diethylether, Metyl-t-butylether oder Tetrahydrofuran verwendet.

3. Verfahren nach Anspruch 1 oder 2, wobei man die Behandlung mit Hydrazin und dem Alkalimetallhydroxid in Anwesenheit von Ethylenglykol, Ethylenglykolmonomethylether oder Diethylenglykol durchführt.

4. Verfahren nach Anspruch 3, wobei man die Behandlung mit Hydrazin und dem Alkalirnethallhydroxid in Anwesenheit von Diethylenglycol durchführt.

5. Verfahren nach einem der vorhergehenden Ansprüche, wobei man das durch die Umsetzung mit Oxalylchlorid erhaltene Produkt zunächst mit Hydrazin und anschließend mit dem Alkalimetallhydroxid behandelt.

6. Verfahren nach Anspruch 5, wobei man die Behandlung mit dem Alkalimetallhydroxid bei einer Temperatur im Bereich von 120 bis 180 °C durchführt.

7. Verfahren nach Anspruch 6, wobei man während der Behandlung die bei der Behandlungstemperatur flüchtigen Bestandteile zumindest teilweise entfernt.

8. Verfahren nach einem der vorhergehenden Ansprüche, wobei man die mittlere Phase vor dem Ansäuern mit einem Gemisch aus Wasser und einem mit Wasser nicht mischbaren Ether versetzt.

9. Verfahren nach Anspruch 8, wobei man das gewünschte Produkt aus der Etherphase gewinnt, indem man der Etherphase einen aliphatischen oder cycloaliphatischen Kohlenwasserstoff zusetzt, der höher siedet als der Ether.

10. Verfahren nach Anspruch 8 oder 9, wobei man zur Gewinnung des gewünschten Produktes den Ether zumindest teilweise abdestilliert.

11. Verfahren nach einem der vorhergehenden Ansprüche, wobei man zur Herstellung der Verbindung der Formel III 2-Benzyl-4,4-dimethyl-1-pyrrolin mit einem ω-Halogen-4-chloracetophenon in einem polaren organischen Lösungsmittel in Anwesenheit eines Alkalimetallhydrogencarbonats und/oder eines Alkalimethallcarbonats in fester Form umsetzt.

12. Verfahren nach Anspruch 11, wobei man als polares organisches Lösungsmittel Methanol verwendet.

13. Verfahren nach Anspruch 11 oder 12, wobei man die Umsetzung in Anwesenheit von festem Natriumhydrogencarbonat durchführt.

14. Verbindung der Formel II

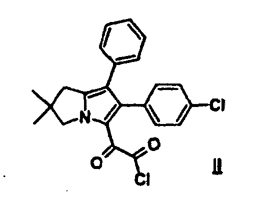

15. Verbindung der Formel I 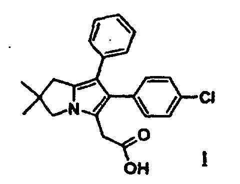 in kristalliner Form mit einem einzigen Endotherm im DSC-Diagramm, der im Bereich von 155 bis 170 °C liegt.

16. Verbindung nach Anspruch 15, die folgende wesentliche Peaks im IR-Spektrum aufweist (Wellenzahl, cm⁻¹): 1706; 1601; 1536; 1487; 1463; 1450; 1441; 1413; 1395; 1383; 1369; 1293; 1219; 1177; 1099; 1013; 836; 765; 698.

17. Verbindung nach Anspruch 15 oder 16 mit folgenden charakteristischen d-Werten (Netzebenenabstand) im Röntgenbeugungsdiagramm: 11,9; 4,2; 4,0.

18. Verbindung nach Anspruch 17 mit folgenden d-Werten: 11,9; 8,2; 7,9; 6,4; 5,9; 5, 8; 5,6; 5,3; 5,0; 4,9; 4,7; 4,5; 4,4; 4,3; 4,2; 4,05;4,0; 3,9; 3,7; 3,6; 3,55; 3,5; 3,4; 3,3; 3,25; 3,2; 3,1; 2,95; 2,9; 2,85; 2,8; 2,75; 2,7; 2,65; 2,6.

19. Pharmazeutisches Mittel, enthaltend die Verbindung gemäß einem der Ansprüche 15 bis 18, ggf. zusammen mit üblichen Hilfsstoffen.

20. Verwendung der Verbindungen gemäß einem der Ansprüche 15 bis 18 zur Herstellung eines pharmazeutischen Mittels zur Behandlung von Erkrankungen des rheumatischen Formenkreises.

21. Verfahren zur Herstellung der Verbindungen der in Ansprüche 15 bis 18, wobei man zu einer etherischen Lösung der Verbindung der Formel I einen aliphatischen oder cycloaliphatischen Kohlenwasserstoff gibt, der höher als der Ether siedet, den Ether gegebenenfalls zumindest teilweise abdestilliert und die Verbindung der Formel I gewinnt.

## Claims

1. Process for the preparation of the compound of the formula I 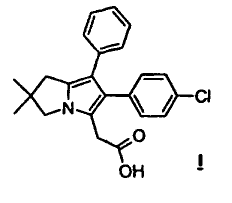 where the compound of the formula III 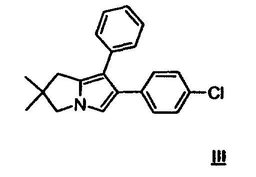 is reacted with oxalyl chloride and the product obtained is treated with hydrazine and an alkali metal hydroxide in the aqueous phase at from 120 to 180°C in the presence of an aliphatic mono- or dialcohol having a boiling point of at least 140°C, after treatment is complete a three-phase system is produced by addition of an ether and the compound of the formula I is obtained by acidifying the middle phase.

2. Process according to Claim 1, the ether used being diethyl ether, methyl t-butyl ether or tetrahydrofuran.

3. Process according to Claim 1 or 2, the treatment with hydrazine and the alkali metal hydroxide being carried out in the presence of an aliphatic mono- or dialcohol.

4. Process according to Claim 3, the treatment with hydrazine and the alkali metal hydroxide being carried out in the presence of diethylene glycol.

5. Process according to one of the preceding Claims, the product obtained by the reaction with oxalyl chloride being treated first with hydrazine and then with the alkali metal hydroxide.

6. Process according to Claim 5, the treatment with the alkali metal hydroxide being carried out at a temperature in the range from 120 to 180°C.

7. Process according to Claim 6, the constituents which are volatile at the treatment temperature being removed at least partially during the treatment.

8. Process according to one of the preceding Claims, the middle phase being treated with a mixture of water and a water-immiscible ether before acidification.

9. Process according to Claim 8, the desired product being obtained from the ether phase by adding to the ether phase an aliphatic or cycloaliphatic hydrocarbon which has a higher boiling point than the ether.

10. Process according to Claim 8 or 9, the ether being at least partially distilled off to obtain the desired product.

11. Process according to one of the preceding Claims, where, for the preparation of the compound of the formula III, 2-benzyl-4,4-dimethyl-1-pyrroline is reacted with an ω-halo-4-chloroacetophenone in a polar organic solvent in the presence of an alkali metal hydrogencarbonate and/or of an alkali metal carbonate in solid form.

12. Process according to Claim 11, the polar organic solvent used being methanol.

13. Process according to Claim 11 or 12, the reaction being carried out in the presence of solid sodium hydrogencarbonate.

14. Compound of the formula II

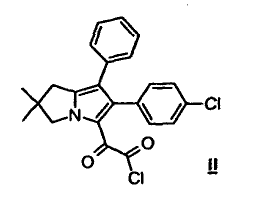

15. Compound of the formula I 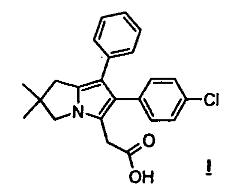 in crystalline form having a single endotherm in the DSC diagram, which is in the range from 155 to 170°C.

16. Compound according to Claim 15, which has the following significant peaks in the IR spectrum (wavenumber, cm⁻¹): 1706; 1601; 1536; 1487; 1463; 1450; 1441; 1413; 1395; 1383; 1369; 1293; 1219; 1177; 1099; 1013; 836; 765; 698.

17. Compound according to Claim 15 or 16 having the following characteristic d values (lattice distance) in the X-ray diffraction diagram: 11.9; 4.2; 4.0.

18. Compound according to Claim 17 having the following d values: 11.9; 8.2; 7.9; 6.4; 5.9; 5. 8; 5.6; 5.3; 5.0; 4.9; 4.7; 4.5; 4.4; 4.3; 4.2; 4.05;4.0; 3.9; 3.7; 3.6; 3.55; 3.5; 3.4; 3.3; 3.25; 3.2; 3.1; 2.95; 2.9; 2.85; 2.8; 2.75; 2.7; 2.65; 2.6.

19. Pharmaceutical composition comprising the compound as claimed in one of Claims 15 to 18, if appropriate together with customary excipients.

20. Use of the compounds as claimed in one of Claims 15 to 18 for the production of a pharmaceutical composition for the treatment of disorders of the rheumatic type.

21. Process for the preparation of the compounds of Claims 15 to 18, where, to an ethereal solution of the compound of the formula I, an aliphatic or cycloaliphatic hydrocarbon is added which boils higher than the ether, the ether is optionally at least partially removed by distillation and the compound of the formula I is obtained.

## Revendications

1. Procédé pour la préparation du composé de la formule I 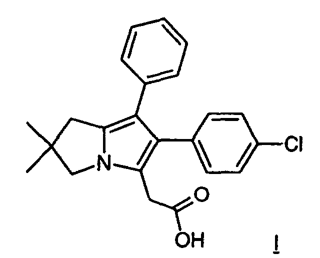 dans lequel on fait réagir le composé de la formule III 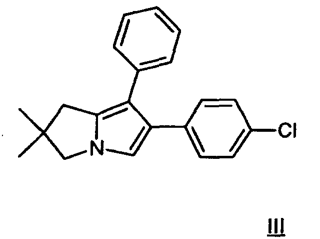 avec du chlorure d'oxalyle et on traite le produit obtenu avec de l'hydrazine et un hydroxyde de métal alcalin en phase aqueuse à de 120 à 180°C en présence d'un mono- ou di-alcool aliphatique avec un point d'ébullition d'au moins 140°C, on produit, après l'achèvement du traitement par addition d'un éther, un système à trois phases et on récupère le composé de la formule I par acidification de la phase du milieu.

2. Procédé selon la revendication 1, dans lequel on utilise comme éther du diéthyléther, du méthyl-t-butyléther ou du tétrahydrofurane.

3. Procédé selon la revendication 1 ou 2, dans lequel on réalise le traitement avec l'hydrazine et l'hydroxyde de métal alcalin en présence d'éthylèneglycol, de monométhyléther d'éthylèneglycol ou de diéthylèneglycol.

4. Procédé selon la revendication 3, dans lequel on réalise le traitement avec l'hydrazine et l'hydroxyde de métal alcalin en présence de diéthylèneglycol.

5. Procédé selon l'une quelconque des revendications précédentes, dans lequel on traite le produit obtenu par la réaction avec le chlorure d'oxalyle tout d'abord avec de l'hydrazine et ensuite avec l'hydroxyde de métal alcalin.

6. Procédé selon la revendication 5, dans lequel on réalise le traitement avec l'hydroxyde de métal alcalin à une température dans le domaine de 120 à 180°C.

7. Procédé selon la revendication 6, dans lequel on élimine au moins partiellement pendant le traitement les parties volatiles à la température du traitement.

8. Procédé selon l'une quelconque des revendications précédentes, dans lequel on ajoute la phase du milieu avant l'acidification avec un mélange constitué d'eau et d'un éther non miscible à l'eau.

9. Procédé selon la revendication 8, dans lequel on récupère le produit souhaité à partir de la phase d'éther en ce que l'on ajoute à la phase d'éther un hydrocarbure aliphatique ou cydoaliphatique qui bout à une température plus élevée que la température d'ébullition de l'éther.

10. Procédé selon la revendication 8 ou 9, dans lequel on élimine au moins partiellement par distillation l'éther pour obtenir le produit souhaité.

11. Procédé selon l'une quelconque des revendications précédentes, dans lequel on fait réagir pour la préparation du composé de la formule III de la 2-benzyl-4,4-diméthyl-1-pyrroline avec une ω-halogéno-4-chloracétophénone dans un solvant organique polaire en présence d'un hydrogénocarbonate de métal alcalin et/ou d'un carbonate de métal alcalin dans une forme solide.

12. Procédé selon la revendication 11, dans lequel on utilise comme solvant organique polaire du méthanol.

13. Procédé selon la revendication 11 ou 12, dans lequel on réalise la réaction en présence d'hydrogénocarbonate de sodium solide.

14. Composé de la formule II

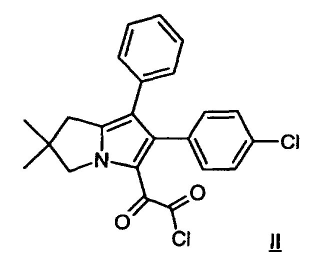

15. Composé de la formule I 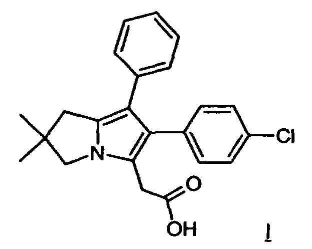 dans une forme cristalline avec un endotherme unique dans le diagramme DSC qui se trouve dans le domaine de 155 à 170°C.

16. Composé selon la revendication 15, qui présente les pics principaux suivants dans le spectre IR (longueur d'onde, cm⁻¹) : 1706 ; 1601 ; 1536 ; 1487 ; 1463 ; 1450 ; 1441 ; 1413 ; 1395 ; 1383 ; 1369 ; 1293 ; 1219 ; 1177 ; 1099 ; 1013 ; 836 ; 765 ; 698.

17. Composé selon la revendication 15 ou 16 avec les valeurs caractéristiques d suivantes (écartement des plans du réseau) dans un diagramme de diffraction aux rayons X : 11,9 ; 4,2 ; 4,0.

18. Composé selon la revendication 17 avec les valeurs d suivantes : 11,9 ; 8,2 ; 7,9 ; 6,4 ; 5,9 ; 5,8 ; 5,6 ; 5,3 ; 5,0 ; 4,9 ; 4,7 ; 4,5 ; 4,4 ; 4,3 ; 4,2 ; 4,05 ; 4,0 ; 3,9 ; 3,7 ; 3,6 ; 3,55 ; 3,5 ; 3,4 ; 3,3 ; 3,25 ; 3,2 ; 3,1 ; 2,95 ; 2,9 ; 2,85 ; 2,8 ; 2,75 ; 2,7 ; 2,65 ; 2,6.

19. Agent pharmaceutique contenant le composé selon l'une quelconque des revendications 15 à 18 éventuellement en association avec des auxiliaires classiques.

20. Utilisation des composés selon l'une quelconque des revendications 15 à 18 pour la préparation d'un agent pharmaceutique destiné au traitement de maladies du type rhumatismal.

21. Procédé pour la préparation des composés des revendications 15 à 18, dans lequel on ajoute à une solution éthérée du composé de la formule I un hydrocarbure aliphatique ou cycloaliphatique qui bout à une température plus élevée que la température d'ébullition de l'éther, on élimine facultativement au moins partiellement l'éther par distillation et on récupère le composé de la formule I.
